(19) Europäisches Patentamt
European Patent Office
Office européen des brevets

(11) Veröffentlichungsnummer : **0 332 918 B1**

(12) # EUROPÄISCHE PATENTSCHRIFT

(45) Veröffentlichungstag der Patentschrift : **29.04.92 Patentblatt 92/18**

(51) Int. Cl.$^5$ : **A61K 45/06,** A61K 31/275, // (A61K31/275, 31:215)

(21) Anmeldenummer : **89103451.4**

(22) Anmeldetag : **28.02.89**

(54) **Erzeugnisse, enthaltend einen Calciumantagonisten und einen Lipidsenker.**

Die Akte enthält technische Angaben, die nach dem Eingang der Anmeldung eingereicht wurden und die nicht in dieser Patentschrift enthalten sind.

(30) Priorität : **10.03.88 DE 3807895**

(43) Veröffentlichungstag der Anmeldung : **20.09.89 Patentblatt 89/38**

(45) Bekanntmachung des Hinweises auf die Patenterteilung : **29.04.92 Patentblatt 92/18**

(84) Benannte Vertragsstaaten : **AT BE CH DE FR GB IT LI NL**

(56) Entgegenhaltungen :
**DIALOG 7018516 Embase 88021867; K.-A. BUNGEROTH: "Sekundarpravention nach Herzinfarkt - welche Medikamentose Behandlung ist heute gesichert?", & FORTSCHR. MED. (DE), 1987, 105/31 (607-611)**
**DIALOG 5664296 Embase 84159962; C.D. FURBERG et al.: "Effect of long-term prophylactic treatment on survival after myocardial infarction", & AM. J. MED. (USA), 1984, 76/6A (76-83)**

(56) Entgegenhaltungen :
**PHARMACEUTICAL NEWS INDEX 88-20781; "New cardiovascular therapies discussed; Anti-arrhythmics MAPHY study criticised heart failure Potassium channel activators Calcium antagonists", & SCRIP WORLD PHARMACEUTICAL NEWS, 1988, NO. 1320, PP. 28,29**
**PHARMACEUTICAL NEWS INDEX 83-20993; "Post-infarction scripts in FRG", & SCRIP WORLD PHARMACEUTICAL NEWS NO. 842, Page 2**
**DIALOG 5736075 Embase 84231741; O. LEISZ et al.: "Treatment of hyperlipoproteinaemia by diet and drugs", & AKTUEL. NEUROL. (DE), 1984, 11/4 (129-133)**

(73) Patentinhaber : **KNOLL AG**
**Knollstrasse 32**
**W-6700 Ludwigshafen (DE)**

(72) Erfinder : **Gremm, Dorothee**
**Werderstrasse 34**
**W-6800 Mannheim (DE)**
Erfinder : **Persiel, Ingetraud, Dr.**
**Feldstrasse 51**
**W-6800 Mannheim-Feudenheim (DE)**

(74) Vertreter : **Karau, Wolfgang, Dr. et al**
**BASF Aktiengesellschaft Carl-Bosch-Strasse 38**
**W-6700 Ludwigshafen (DE)**

EP 0 332 918 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es ist bekannt, daß Calciumantagonisten wirksame Pharmaka zur Behandlung der koronaren Herzerkrankung sind (H. Eichstädt "Calcium-Antagonisten", Kapitel 33 aus "Handbuch der inneren Medizin, Band IX/3: Koronarerkrankungen", hrsg. von H. Roskamm, Springer-Verlag, Berlin, 1984; U. Borchard "Spektrum Koronartherapeutika", Aesopus Verlag, Zug, 1985).

Weiter sind bereits eine Reihe von Lipidsenkern beschrieben worden, mit deren Hilfe Hyperlipidämien behandelt werden können (W. Kruse et al "Spektrum Lipidsenker", Aesopus Verlag, Zug, 1985). Von Fibraten ist auch bekannt, daß sie die Fließeigenschaften des Blutes verbessern (z.B. Ernst/Matrai, Therapiewoche 38, 136-139, 1988; Vortrag von Leschke während des Expertengespräches "Aktueller Stand der Therapie von Fettstoffwechselstörungen", Wiesbaden, 10.10.87; Ärzte-Zeitung 6, 29, 1987).

Es wurde nun gefunden, daß sich die Wirkung von Calciumantagonisten durch Zugabe von Lipidsenkern verbessern läßt.

Gegenstand der Erfindung sind Erzeugnisse, enthaltend einen Calciumantagonisten und einen Lipidsenker.

Die Erzeugnisse können als Kombinationspräparate zur gleichzeitigen Anwendung in fixer Kombination oder getrennt zur gleichzeitigen oder zeitlich abgestuften Anwendung vorliegen.

Als Calciumantagonisten seien insbesondere genannt: Diltiazem, Etafenon, Fendilin, Gallopamil, Nifedipin, Prenylamin, Perhexilin und Verapamil. Hiervon sind Verapamil und Gallopamil bevorzugt.

Als Lipidsenker kommen beispielsweise in Betracht: Bezafibrat, Clofibrat sowie Aluminium- und Etofyllinclofibrat, Colestipol, Colestyramin, Dextrothyroxin, Etiroxat, Etofibrat, Fenofibrat, Gemfibrozil, Inositolnicotinat, Magnesium-pyridoxal-5′-phosphat-glutaminat, Probucol, 3-Pyridylmethanol, β-Sitosterin und Xantinolnicotinat. Von diesen Substanzen sind die Fibrate (Bezafibrat, Clofibrat, Etofibrat und Fenofibrat) bevorzugt.

Sofern die Substanzen basische Gruppen enthalten, können sie auch in Form ihrer Salze mit physiologisch verträglichen Säuren für das Kombinationspräparat verwendet werden. Als physiologisch verträgliche Säuren kommen vorzugsweise in Betracht: Salzsäure, Schwefelsäure, Phosphorsäure, Essigsäure, Zitronensäure, Malonsäure, Salicylsäure, Maleinsäure, Fumarsäure, Bernsteinsäure, Ascorbinsäure, Äpfelsäure, Methansulfonsäure, Milchsäure, Gluconsäure, Glucuronsäure, Amidosulfonsäure, Benzoesäure, Weinsäure.

Calciumantagonist und Lipidsenker liegen in der Mischung in Mengen vor, die an der unteren Grenze der normalerweise für diese Substanzen empfohlenen Dosiermengen oder etwas darunter liegen. In der Regel liegt das Verhältnis der Mengen Lipidsenker zu Calciumantagonist im Bereich von 10:1 bis 1:10. Für eine Kombination aus Verapamil und Fenofibrat liegt dieses Verhältnis vorzugsweise bei 1:2 bis 2:1.

Die neue Kombination verbessert die Fließeigenschaften des Blutes im Vergleich zu den Einzelbestandteilen der Kombination in unerwarteter überadditiver Stärke. Sie eignet sich daher zur Therapie und Prophylaxe von Symptomen, die durch Störungen der Myokarddurchblutung bei koronarer Herzerkrankung bedingt sind, d.h. aller Angina-pectoris-Formen, zur Nachbehandlung des Herzinfarktes, zur Anwendung vor und nach Ballondilatation und Bypassoperation.

Die erfindungsgemäßen Erzeugnisse können parenteral, vorzugsweise aber auch oral verabfolgt werden. Zur oralen Applikation eignen sich insbesondere Tabletten, Dragées und Kapseln. Besonders geeignet sind Retardformen. Für die Herstellung der genannten Formen eignen sich die bekannten Verfahren, wie sie beispielsweise in H. Sucker et al, Pharmazeutische Technologie, Thieme-Verlag, Stuttgart 1978 beschrieben sind. Es ist auch möglich, die Einzelbestandteile der Mischung zu Applikationsformen zu verarbeiten und diese zusammen zu verpacken, beispielsweise in einer Durchdrückpackung.

Beispiel 1

20 Retardtabletten Isoptin®RR und 20 Retardkapseln Normalip® werden jeweils paarweise in eine Durchdrückpackung gefüllt.

Beispiel 2

Es wurden Pellets folgender Zusammensetzung hergestellt:

| | | |
|---|---|---|
| a) | Fenofibrat | 100,00 mg |
| | Saccharose | 17,92 mg |
| | Maisstärke | 5,96 mg |
| | Polyvidon | 0,74 mg |
| | Talkum | 1,49 mg |
| | Polymethacrylsäure L* | 1,44 mg |
| | Polymethacrylsäure E* | 0,40 mg |
| | Stearinsäure | 0,05 mg |
| | | 128,00 mg |

* Hersteller: Fa. Röhm, Darmstadt

| | | |
|---|---|---|
| b) | Verapamilhydrochlorid | 100,00 mg |
| | kolloidales Siliziumdioxid | 1,00 mg |
| | Maisstärke | 6,96 mg |
| | Zuckerpellets | 22,65 mg |
| | Ethylcellulose | 3,40 mg |
| | Glycerin-mono/di-oleat | 2,27 mg |
| | ®Eudragit RS** | 1,45 mg |
| | ®Eudragit S*** | 2,90 mg |
| | Dibutylphthalat | 0,29 mg |
| | Talkum | 9,38 mg |
| | | 150,30 mg |

** Polymerisat aus Acryl- und Metharylsäureestern mit einem geringen Gehalt an quaternären Ammoniumgruppen (Hersteller: Fa. Röhm, Darmstadt)

*** Anionisches Polymerisat aus Methacrylsäure und Methacrylsäureestern (Hersteller: Fa. Röhm, Darmstadt)

Es wurden jeweils 128,0 mg Pellets a) und 150,3 mg Pellets b) in Steckkapseln gefüllt.

Beispiel 3

Das Beispiel 2 wurde wiederholt, jedoch wurden 192,0 mg Pellets a) und 150,3 mg Pellets b) pro Steckkapsel abgefüllt.

Beispiel 4

Das Beispiel 2 wurde wiederholt, jedoch wurden 128,0 mg Pellets a) und 300,6 mg Pellets b) pro Steckkapsel abgefüllt.

Beispiel 5

Es wurden Tabletten folgender Zusammensetzung gepreßt:

| | | |
|---|---|---|
| a) | Fenofibrat | 80,00 |
| | Saccharose | 14,35 |
| | Maisstärke | 4,77 |
| | Polyvidon | 0,59 |
| | Talkum | 1,19 |

| | | |
|---|---|---|
| b) | Verapamilhydrochlorid | 80,00 |
| | kolloidales Siliziumdioxid | 0,80 |
| | Maisstärke | 5,60 |
| | Zuckerpellets | 18,17 |
| | Ethylcellulose | 2,72 |
| | Glycerin-mono/di-oleat | 1,81 |
| | Talkum | 7,50 |

Die Tabletten wurden im Verhältnis 1:1 in Kapseln gefüllt.

## Patentansprüche

1. Erzeugnisse, enthaltend einen Calciumantagonisten und einen Lipidsenker.

2. Erzeugnisse gemäß Anspruch 1 als Kombinationspräparat zur gleichzeitigen Anwendung in fixer Kombination oder getrennt zur gleichzeitigen oder zeitlich abgestuften Anwendung.

3. Erzeugnisse gemäß Anspruch 1, dadurch gekennzeichnet, daß das Verhältnis von Calciumantagonist zu Lipidsenker bei 10:1 bis 1:10 liegt.

4. Erzeugnisse gemäß Anspruch 1, dadurch gekennzeichnet, daß der Calciumantagonist Verapamil und der Lipidsenker Fenofibrat ist.

5. Erzeugnis gemäß Anspruch 4, dadurch gekennzeichnet, daß das Verhältnis Verapamil zu Fenofibrat 1:2 bis 2:1 ist.

6. Erzeugnisse gemäß Anspruch 1, zur Verwendung bei der Bekämpfung von Krankheiten.

7. Verwendung der Erzeugnisse gemäß Anspruch 1 zur Herstellung von Arzneimitteln zur Behandlung der koronaren Herzerkrankung.

## Revendications

1. Produits contenant un antagoniste du calcium et un abaisseur de lipide.

2. Produits selon la revendication 1 comme préparation de combinaison pour utilisation sumultanée en combinaison fixe ou séparée pour utilisation simultanée ou échelonnée dans le temps.

3. Produits selon la revendication 1, caractérisé par le fait que le rapport de l'antagoniste du calcium à l'abaisseur de lipide est compris entre 10/1 et 1/10.

4. Produits selon la revendication 1, caractérisé par le fait que l'antagoniste du calcium est le Verapamil et l'abaisseur de lipide est le Fenofibrate.

5. Produits selon la revendication 4, caractérisé par le fait que le rapport du Verapamil au Fenofibrate est de 1/2 à 2/1.

6. Produit selon la revendication 1 pour utilisation pour la lutte contre les maladies.

7. Utilisation des produits selon la revendication 1 pour la préparation de médicaments pour le traitement des maladies du coeur coronariennes.

## Claims

1. A product containing a calcium antagonist and a lipid-lowering agent.

2. A product as claimed in claim 1, as a combination preparation for simultaneous use in a fixed combination or as separate components for use simultaneously or sequentially.

3. A product as claimed in claim 1, wherein the ratio of calcium antagonist to lipid-lowering agent is from 10 : 1 to 1 : 10.

4. A product as claimed in claim 1, wherein the calcium antagonist is verapamil and the lipid-lowering agent is fenofibrate.

5. A product as claimed in claim 4, wherein the ratio of verapamil to fenofibrate is from 1 : 2 to 2 : 1.

6. A product as claimed in claim 1, for use to control diseases.

7. Use of a product as claimed in claim 1 for the preparation of drugs for the treatment of coronary heart disease.